# FASCICULE DE BREVET EUROPEEN

(11) **EP 0 688 328 B1**
(45) Date de publication et mention de la délivrance du brevet: **12.02.1997**
(21) Numéro de dépôt: 94909171.4
(22) Date de dépôt: 10.03.1994
(51) Int. Cl.: C07H 15/04

(54) **NOUVEAU PROCEDE DE PREPARATION D'ALKYLPOLYOSIDES FAIBLEMENT COLORES ET A FAIBLE TENEUR EN PRODUITS DE DEGRADATION ACIDE**
NEUES VERFAHREN ZUR HERSTELLUNG VON SCHWACH GEFÄRSTEN ALKYLPOLYOSIDEN UND MITGERINGEM GEHALT IN SAUREN ABBAUPRODUKTEN
NOVEL METHOD FOR PREPARING SLIGHTLY COLOURED ALKYLPOLYSACCHARIDES HAVING A LOW ACID BREAKDOWN PRODUCT CONTENT

(30) Priorité: 12.03.1993 FR 9302877
(43) Date de publication de la demande: 27.12.1995
(73) Titulaire: SOCIETE D'EXPLOITATION DE PRODUITS POUR LES INDUSTRIES CHIMIQUES-SEPPIC, F-75321 Paris Cédex 07 (FR)
(72) Inventeur: BOITEUX, Jean-Pierre, F-81710 Saix (FR); ROLLAND, Hervé, F-81100 Castres (FR)
(74) Mandataire: Hubert, Philippe
(86) Numéro de dépôt international: FR9400263
(87) Numéro de publication internationale: WO9420513

(56) Documents cités:
- EP-A- 0 132 046
- EP-A- 0 411 980

## Description

La présente invention a essentiellement pour objet un nouveau procédé de préparation d'alkylpolyosides faiblement colorés et à faible teneur en produits de dégradation acide obtenus par réaction d'un saccharide avec un alcool gras en excès, en présence d'un catalyseur acide, suivie d'une neutralisation avec une base.

Les alkylpolyosides sont des agents de surface non-ioniques, déjà utilisés dans une large gamme d'applications industrielles.

La préparation d'alkylpolyosides à partir d'un sucre réducteur et d'un alcool gras a été largement décrite dans la littérature.

Les procédés les plus couramment utilisés font intervenir la réaction, en présence d'un catalyseur acide, entre un monosaccharide (ou une source de monosaccharide) et un alcool gras.

Une variante de ces procédés consiste à opérer par transéthérification, c'est-à-dire à mettre en oeuvre, préalablement ou en même temps que l'alcool gras, un alcool léger, comme par exemple le méthanol ou le butanol, pour obtenir un alkylpolyoside à chaîne courte qui réagit avec l'alcool gras pour conduire à un alkylpolyoside à chaîne longue.

Dans tous les cas, la réaction est réalisée avec un excès molaire d'alcool gras (généralement 2 à 8 moles d'alcool gras par unité de monosaccharide) de telle sorte qu'il est ensuite nécessaire d'éliminer l'alcool en excès.

L'élimination de l'alcool gras en excès est généralement réalisée par distillation sous vide.

Cependant, les alcools mis en oeuvre ayant des points d'ébullition élevés (de l'ordre de 120°C à 220°C sous des pressions résiduelles comprises entre 0,5 et 20 mm de mercure), la distillation doit être réalisée à une température relativement élevée, ce qui peut engendrer des problèmes quant à la couleur des produits obtenus.

Pour limiter les risques de coloration, lors de l'élimination de l'alcool, la méthode industrielle actuellement la plus satisfaisante consiste à employer un évaporateur à film tombant, à film tournant ou un évaporateur flash. Cette technique, dont le principal avantage est de diminuer les temps de surchauffe des produits a par exemple été décrite dans les documents US 3,565,885 et EP0418458.

Au cours de la phase d'élimination de l'alcool, outre la température, le pH du milieu réactionnel est un autre paramètre influençant la coloration finale des produits.

Avant la distillation de l'alcool, il est indispensable de neutraliser les restes de catalyseur présents dans le produit de réaction. Cette neutralisation doit être effectuée dans une zone de pH optimum. Ainsi, un pH alcalin favorise la dégradation des sucres résiduels présents dans le milieu réactionnel avec, notamment la formation de produits de polycondensation aldolique très fortement colorés. Tandis qu'une distillation à un pH trop acide, bénéfique au niveau de la coloration, se fait au détriment de la stabilité à l'hydrolyse des alkylpolyosides. En milieu acide, le glucose, résiduel ou formé par hydrolyse, est anhydrisé en 5-hydroxyméthyl-furfural, lequel peut donner par dégradation des acides tels que l'acide formique ou l'acide lévulinique. On trouve également dans certains alkylpolyosides commerciaux des traces d'acide acétique, lactique ou glycolique.

La neutralisation du catalyseur est généralement réalisée au moyen d'une base minérale forte telle que l'hydroxyde de sodium ou de potassium, ce qui, compte tenu du caractère anhydre du milieu, accroît les difficultés d'ajustement du pH.

Pour remédier à ces inconvénients, divers agents de neutralisation ont été proposés.

C'est ainsi que l'on peut citer :
- les résines échangeuses d'ions (US 3,565,885) ;
- les alcoolates alcalins et alcalino-terreux, en particulier les alcoolates d'aluminium (EP 0132046) ;
- les sels d'acides faibles et de bases fortes comme le carbonate de sodium ou l'acétate de sodium (US 4,939,245).

Toutes les solutions proposées jusqu'à présent permettent effectivement d'améliorer la coloration des produits finis.

Cependant, ces solutions s'avèrent insuffisantes lorsque l'on souhaite obtenir des produits très faiblement colorés.

C'est pourquoi il a été proposé de recourir en outre à des procédés plus ou moins complexes de décoloration et/ou de stabilisation des couleurs :
- traitement par l'ozone (DE 3910269 et WO 91/090043) ;
- stabilisation des couleurs par le métabisulfite de sodium (US 4,557,729) ;
- décoloration par l'utilisation de charbons actifs (EP 306 650) ;

Des procédés permettant de limiter la formation de produits colorés, en éliminant, en amont, les sucres résiduels du mélange réactionnel, ont également été proposés :
- hydrogénation catalytique (US 4,904,774) ;
- traitement au borohydrure de sodium (EP 387 916 et EP 388 857).

La présente invention a pour but de résoudre le problème technique consistant en la fourniture d'un nouveau procédé de préparation d'alkylpolyosides qui conduise à des produits très faiblement colorés ou incolores et renfermant une quantité réduite de produits de dégradation acide sans recourir à des procédés complémentaires d'élimination de sucres résiduels, de décoloration et/ou de stabilisation des couleurs.

La présente invention a également pour but de fournir un nouveau procédé répondant aux exigences précitées, et qui puisse être mis en oeuvre de façon aisée et peu coûteuse à l'échelle industrielle.

La solution conforme à la présente invention, pour résoudre ce problème technique consiste en un procédé de préparation d'un alkylpolyoside comportant de 6 à 32 atomes de carbone dans sa chaîne alkyle, du type comprenant :
a) la réaction, en présence d'un catalyseur acide entre un monosaccharide ou une source de monosaccharide et
   - soit, un alcool gras ayant une chaîne hydrocarbonée comportant au moins 6 atomes de carbone ;
   - soit, un alcool inférieur de formule CₙH₂ₙ₊₁OH, dans laquelle n est un nombre entier compris entre 1 et 5 ; et un alcool gras ayant une chaîne hydrocarbonée comportant au moins 6 atomes de carbone ;
b) la neutralisation du catalyseur utilisé à l'étape a) et
c) l'élimination de l'alcool gras en excès ;
caractérisé en ce que l'agent de neutralisation utilisé à l'étape b) précitée est une amine tertiaire de formules : 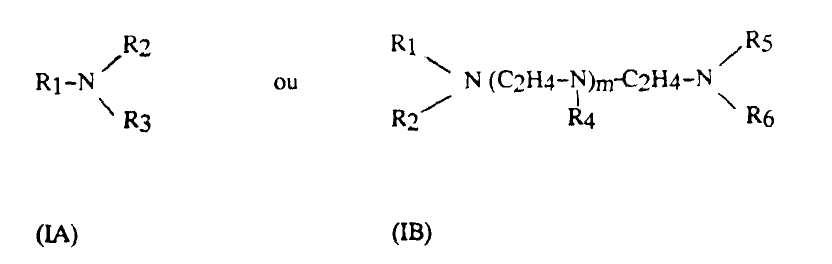 dans lesquelles ;
- m représente un nombre entier compris entre 0 et 5;
- R₁, R₂, R₃, R₄, R₅, R₆ identiques ou différents représentent indépendamment :
   un radical alkyle ayant de 1 à 6 atomes de carbone ;
   un radical hydroxyalkyle ou dihydroxyalkyle ayant de 2 à 4 atomes de carbone ;
   un radical phényle ;
   un radical de formule (CₙH₂ₙ-O)ₓH, dans laquelle n est un nombre entier compris entre 2 et 4 et x est un nombre entier compris entre 2 et 5 ;
- R₁R₂ pris ensemble avec l'atome d'azote auquel ils sont rattachés, pouvant représenter un hétérocycle, saturé ou non, substitué ou non, de 5 à 7 éléments et comportant de 1 à 3 hétéroatomes choisis parmi l'oxygène, l'azote et le soufre.

La présente invention est en effet fondée sur la découverte surprenante et inattendue du fait que l'utilisation de bases aminées tertiaires de formules (IA) et (IB) telles que définies précédemment, dans la neutralisation des catalyseurs d'éthérification, permettait d'améliorer considérablement la qualité des alkylpolyosides obtenus en ce qui concerne notamment la coloration et la teneur en produits de dégradation acide.

Ces bases aminées tertiaires sont inertes vis-à-vis des sucres réducteurs utilisés, et l'absence d'atome d'hydrogène sur l'azote élimine la possibilité de réaction secondaire génératrice de sous-produits colorés.

En outre, la présence d'un doublet libre sur l'atome d'azote permet une neutralisation du milieu par formation d'un dérivé d'ammonium quaternaire et conduit à des pH beaucoup plus stables qu'avec les bases minérales ou organiques utilisées jusqu'à présent, aussi bien au cours de l'étape de neutralisation proprement dite que durant la phase de distillation de l'alcool gras.

Enfin, en raison d'une meilleure compatibilité avec le milieu réactionnel, l'ajustement du pH est fortement facilité et de ce fait, l'optimisation de ce dernier lors de la distillation devient beaucoup moins critique qu'avec les agents de neutralisation utilisés jusqu'à présent. L'acidité des produits obtenus est en outre nettement plus faible.

Les composés de formules IA préférés selon l'invention sont ceux dans lesquels l'un au moins de R₁, R₂ et R₃ représente un radical hydroxyalkyle ou dihydroxyalkyle ayant de 2 à 4 atomes de carbone.

Parmi ces composés préférés, les plus intéressants sont ceux choisis parmi la triéthanolamine, la triisopropanolamine, la N-méthyl diéthanolamine ; la N,N-diméthyl éthanolamine, la N,N-diméthyl propanolamine, la N,N-diéthyl dihydroxypropylamine, la N,N-dibutyl éthanolamine.

Une autre classe préférée de composés de formule IA selon l'invention comprend les composés dans lesquels R₁ R₂, pris ensemble avec l'atome d'azote auquel ils sont rattachés, forment un hétérocycle et R₃ représente un radical alkyle ayant de 1 à 6 atomes de carbone ou un radical hydroxyalkyle ayant de 2 à 4 atomes de carbone.

Parmi ces hétérocycles azotés préférés, on citera le N-méthyl pyrrole, la N-méthyl pyrrolidone, la N-méthyl pyrrolidine, la N-méthyl piperidine, la N-méthyl morpholine, la N-hydroxyéthyl morpholine.

Les composés de formule IB préférés selon l'invention sont ceux dans lesquels m est égal à 0 et l'un au moins de R₁, R₂, R₅ et R₆ représente un radical hydroxyalkyle ayant de 2 à 4 atomes de carbone.

Parmi ces composés préférés, on citera la N,N,N',N'-tétrakis(hydroxypropyl) éthylène diamine.

Il est à noter que tous les composés précités ont l'avantage d'être parfaitement connus et ne présentent aucun inconvénient sur le plan toxicologique, puisqu'ils sont notamment employés couramment dans des produits d'hygiène corporelle.

Ainsi de tels composés peuvent être utilisés sans inconvénient notable et il n'est pas nécessaire de les éliminer des produits finis.

Il en est par exemple ainsi de la triéthanolamine ou de la N,N,N',N'-tétrakis(hydroxypropyl) éthylène diamine.

Dans le procédé conforme à la présente invention, les étapes a et c sont classiques et l'homme de métier pourra se reporter à la littérature et notamment aux nombreux documents précités, pour déterminer les conditions réactionnelles les mieux adaptées.

L'alcool gras utilisé à l'étape a) peut être représenté par la formule R(OR')_{y}OH dans laquelle R représente un radical alkyle, alcényle ou alkylphényle à chaîne droite ou ramifiée ayant de 6 à 22 atomes de carbone ; R' représente un groupe alkylène ayant de 2 à 4 atomes de carbone et y représente un nombre compris entre 0 et 20.

Les alcools suceptibles d'être utilisés incluent les alcools linéaires ou ramifiés tels que : l'hexanol, heptanol, octanol, nonanol, décanol, dodécanol, tridécanol, tétradécanol, pentadécanol, hexadécanol, heptadécanol, octadécanol, méthylpentanol, méthylhexanol, méthylheptanol, méthyloctanol, méthylnonanol, méthyldécanol, méthylundécanol, méthyltridécanol, méthyltétradécanol, méthyl pentadécanol, méthylpentadécanol, méthylhexadécanol, méthylheptadécanol, éthylhexanol, éthyloctanol, éthyldécanol, éthyldodécanol, 2-heptanol, 2-nonanol, 2-undécanol, 2-tridécanol, 2-pentadécanol, 2-heptadécanol, 2-butyloctanol, 2-hexyloctanol, 2-octyloctanol, 2-hexyldécanol et le 2-octyldécanol ; les alcénols tels que l'hexènol, heptènol, octènol, nonènol, décènol, undécènol, dodécènol, tridécènol, tetradécènol, pentadécènol, hexadécènol, heptadécènol, heptadécènol et l'octadécènol ; les alkyphénols tels que l'octylphénol et le nonylphénol ; et les arylalcools tels que par exemple l'alcool benzylique.

Ces alcools ou leurs adduits alkoxylés peuvent être utilisés seuls ou en mélange.

Les saccharides utilisés dans le procédé conforme à la présente invention sont des monosaccharides , des disaccharides, des oligosaccharides ou des polysaccharides.

Parmi les monosaccharides susceptibles d'être utilisés, on citera le glucose, le mannose, le galactose, l'allose, l'altrose, le gulose ou dextrose, l'idose, le talose, l'arabinose, le ribose, le xylose et le lyxose ; le glucose étant particulièrement préféré pour des raisons de disponibilité et de faible coût.

Parmi les disaccharides et les oligosaccharides suceptibles d'être utilisés, on citera le maltose, le maltotriose, le lactose, le saccharose, le gentobiose et le cellobiose.

Parmi les polysaccharides suceptibles d'être utilisés, on citera la cellulose, l'amidon, l'amylopectine, l'hémicellulose, l'inuline, le dextrone, la dextrine, le xylane et les sirops de glucose.

La quantité d'alcool et de saccharide utilisée à l'étape a, sera en général telle que le rapport molaire de l'alcool au monomère d'ose soit de 1:1 à 8:1.

Le catalyseur acide utilisé à l'étape a, peut être choisi parmi ceux généralement connus dans la technique en particulier l'acide sulfurique, l'acide paratoluènesulfonique, l'acide chlorhydrique, l'acide phosphorique, l'acide phosphoreux, l'acide hypophosphoreux et leurs mélanges.

La quantité de catalyseur acide utilisé sera d'environ 0,001 à 0,05 mmole par mmole de monomère d'ose.

Lors de l'étape a, la température réactionnelle sera généralement comprise entre environ 90 et environ 120°C et la durée de la réaction entre environ 3 et environ 6 heures.

La quantité d'agent de neutralisation utilisée à l'étape b) dans le procédé de la présente invention doit être suffisante pour permettre l'obtention d'un pH compris entre 5 et 9.

L'agent de neutralisation peut être ajouté au mélange réactionnel sous la forme d'une poudre, ou d'une solution ou dispersion dans un solvant.

Après la neutralisation du catalyseur acide, il est souhaitable d'éliminer l'alcool en excès par distillation. Ceci peut être fait de façon classique, par exemple dans un évaporateur à film à une température de 170 à 200°C sous une pression résiduelle de 1 à 10 mm de mercure.

L'invention sera illustrée par les exemples non limitatifs suivants donnés uniquement à titre illustratif.

Dans ces exemples tous les pourcentages sont exprimés en poids, sauf indications contraires.

### Exemples 1 à 6

Dans un réacteur polyvalent d'une capacité de 120 litres équipé d'un condenseur et d'un ballon récepteur, on introduit 48 kg d'alcool gras ayant un poids moléculaire d'environ 165 et présentant la composition moyenne suivante :

C₁₀H₂₁OH : 85 %

C₁₂H₂₅OH : 12 %

C₁₄H₂₉OH : 3 %

Après addition de 13,1 kg de glucose anhydre on porte la température du mélange maintenu sous agitation à 90°C.

Le catalyseur acide, constitué d'un mélange comprenant 0,095 kg d'acide sulfurique à 98 % et 0,065 kg d'acide hypophosphoreux à 50 %, est alors introduit dans le réacteur.

Le mélange réactionnel est porté à une température de 105°C sous une pression résiduelle de 15 mm de mercure afin d'éliminer l'eau provenant de la formation de l'alkylpolyoside.

Ces conditions de température et de pression sont maintenues pendant trois heures, et le taux de transformation du glucose en alkylpolyoside atteint une valeur de 97 %.

Le milieu est alors refroidi à 45°C.

Le produit réactionnel ainsi obtenu est partagé en plusieurs fractions pour des essais comparatifs de neutralisation.

Sur chacune des fractions obtenues après neutralisation, l'alcool gras en excès est éliminé par distillation sur un évaporateur à film.

L'alkylpolyoside obtenu est repris par l'eau pour obtenir une solution à 55 % environ de matière sèche.

On a rassemblé dans le tableau 1 ci-après les résultats obtenus en mentionnant, pour chaque exemple, la nature de l'agent de neutralisation utilisé, le pH de neutralisation ainsi que les principales caractéristiques des produits réactionnels obtenus.

**TABLEAU 1**

| **EXEMPLE** | **BASE UTILISEE** | **pH DE NEUTRALISATION SUR DILUTION A 1%** | **PRODUIT FINI-pH PRODUIT COLORATION VCS, MATIERE SECHE** |
|---|---|---|---|
| 1 | TRIETHANOLAMINE | pH 1% : 8,3 | pH : 7,5 |
| | | | COLORATION : 5 |
| | | | MATIERE SECHE : 54,9 % |
| 2 | TRIETHANOLAMINE | pH 1% : 7,5 | pH : 6,75 |
| | | | COLORATION : 5- |
| | | | MATIERE SECHE : 54,4 % |
| 3 | N,N,N',N'-tétrakis | pH 1% : 5,65 | pH : 5,25 |
| | (hydroxypropyl)éthylène diamine | | COLORATION : 5+ |
| | | | MATIERE SECHE : 55,1 % |
| 4 | SOUDE | pH 1% : 7,95 | pH : 5.0 |
| | | | COLORATION : 15 |
| | | | MATIERE SECHE : 54,6 % |
| 5 | DIETHYLENE | pH 1% : 8,8 | pH : 2,9 |
| | GLYCOLAMINE | | COLORATION : 17 |
| | | | MATIERE SECHE : 55,0 % |
| 6 | ACETATE DE SODIUM | pH 1% : 5,55 | pH : 7,0 |
| | | | COLORATION : 18 |
| | | | MATIERE SECHE : 55,0 % |

### Exemple 7 à 9

Un essai analogue a été réalisé en reprenant le protocole expérimental décrit précédemment jusqu'au stade de la neutralisation, et en utilisant 0,111 kg d'acide sulfurique comme catalyseur acide, pour respectivement 15,35 kg de glucose anhydre et 56,30 kg d'acool gras.

Après trois heures de réaction, le taux de transformation du glucose atteint 96 %.

Le milieu est alors refroidi à 45°C.

Le produit réactionnel ainsi obtenu est partagé en plusieurs fractions pour des essais comparatifs de neutralisation.

Sur chacune des fractions obtenues après neutralisation, l'alcool gras en excès est éliminé par distillation sur un évaporateur à film. L'alkylpolyoside obtenu est repris par l'eau pour obtenir une solution à 55 % environ de matière sèche.

On a rassemblé dans le tableau 2 ci-après les résultats obtenus en mentionnant, pour chaque exemple, la nature de l'agent de neutralisation utilisé, le pH de neutralisation ainsi que les principales caractéristiques des produits réactionnels obtenus.

**TABLEAU 2**

| **EXEMPLE** | **BASE UTILISEE** | **pH DE NEUTRALISATION SUR DILUTION A 1%** | **PRODUIT FINI-pH PRODUIT COLORATION VCS, MATIERE SECHE** |
|---|---|---|---|
| 7 | TRIETHANOLAMINE | pH 1% : 5,70 | pH : 6,25 |
| | | | COLORATION : 4 |
| | | | MATIERE SECHE : 57,3 % |
| 8 | N,N,N',N'-tétrakis | pH 1% : 5,25 | pH : 4,70 |
| | (hydroxypropyl)éthylène | | COLORATION : 5- |
| | diamine | | MATIERE SECHE : 56,2 % |
| 9 | SOUDE | pH 1% : 5,35 | pH : 2,60 |
| | | | COLORATION : 4 |
| | | | MATIERE SECHE : 57,5 % |

Les exemples comparatifs donnés précédemment montrent clairement que l'utilisation des amines tertiaires de formule IA et IB dans la neutralisation des catalyseurs d'éthérification, permet d'améliorer considérablement la qualité des alkylpolyosides obtenus en ce qui concerne la coloration et la limitation de formation de produits de dégradation acide (stabilité du pH pendant la distillation) notamment vis-à-vis des agents de neutralisation habituellement utilisés comme l'hydroxyde de sodium, l'acétate de sodium ou des amines primaires comme la diéthylène glycolamine.

### Exemple 10

Un essai analogue à ceux réalisés précédemment a été conduit jusqu'au stade de la neutralisation en utilisant 0,026 kg de chlorhydrine sulfurique (ClSO₃H), pour respectivement 3,12 kg de glucose anhydre 11,44 kg d'alcool gras.

Après 3 heures de réaction, le taux de transformation du glucose atteint 96 %.

Le milieu réactionel, refroidi à 45°C, est neutralisé par la triéthanolamine ; le pH de neutralisation obtenu, mesuré en dilution à 1 %, étant de 6,60.

Après distillation et mise en solution effectuée comme dans les essais précédents, le produit obtenu a les caractéristiques physico-chimiques suivantes :
- pH sur produit 6,10
- Coloration VCS 3
- Matière sèche 55,4 %

## Revendications

1. Procédé de préparation d'un alkylpolyoside comportant de 6 à 32 atomes de carbone dans sa chaîne alkyle, du type comprenant :
a) la réaction, en présence d'un catalyseur acide entre un saccharide ou une source de saccharide et
- soit, un alcool gras ayant une chaîne hydrocarbonée comportant au moins 6 atomes de carbone ;
- soit, un alcool inférieur de formule CₙH₂ₙ₊₁OH, dans laquelle n est un nombre entier compris entre 1 et 5 ; et un alcool gras ayant une chaîne hydrocarbonée comportant au moins 6 atomes de carbone ;
b) la neutralisation du catalyseur utilisé à l'étape a) et
c) l'élimination de l'alcool gras en excès ;
caractérisé en ce que l'agent de neutralisation utilisé à l'étape b) précitée est une amine tertiaire de formules : 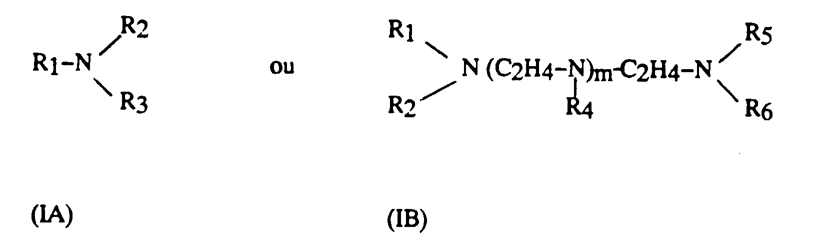 dans lesquelles ;
- m représente un nombre entier compris entre 0 et 5 ;
- R₁, R₂, R₃, R₄, R₅, R₆ identiques ou différents représentent indépendamment :
un radical alkyle ayant de 1 à 6 atomes de carbone ;
un radical hydroxyalkyle ou dihydroxyalkyle ayant de 2 à 4 atomes de carbone ;
un radical phényle ;
un radical de formule (CₙH₂ₙ-O)ₓH, dans laquelle n est un nombre entier compris entre 2 et 4 et x est un nombre entier compris entre 2 et 5 ;
- R₁R₂ pris ensemble avec l'atome d'azote auquel ils sont rattachés, pouvant représenter un hétérocycle, saturé ou non, substitué ou non, de 5 à 7 éléments et comportant de 1 à 3 hétéroatomes choisis parmi l'oxygène, l'azote et le soufre.

2. Procédé selon la revendication 1, caractérisé en ce que l'agent de neutralisation précité est une amine tertiaire de formule IA dans laquelle l'un au moins de R₁, R₂ et R₃ représente un radical hydroxyalkyle ou dihydroxyalkyle ayant de 2 à 4 atomes de carbone.

3. Procédé selon la revendication 2, caractérisé en ce que l'agent de neutralisation est la triéthanolamine.

4. Procédé selon la revendication 1, caractérisé en ce que l'agent de neutralisation précité est une amine tertiaire hétérocyclique de formule IA, dans laquelle R₁ R₂ pris ensemble avec l'atome d'azote auquel ils sont rattachés forment un hétérocycle choisi parmi le pyrrole, la pyrrolidone, la pyrrolidinc, la pipéridine et la morpholine ; et R₃ représente un radical alkyle ayant de 1 à 6 atomes de carbone ou un radical hydroxyalkyle ayant de 2 à 4 atomes de carbone.

5. Procédé selon la revendication 4, caractérisé en ce que l'agent de neutralisation précité est choisi parmi le N-méthyl pyrrole, la N-méthyl pyrrolidone, la N-méthyl pyrrolidine, la N-méthyl piperidine, la N-méthyl morpholine, la N-hydroxyéthyl morpholine.

6. Procédé selon la revendication 1, caractérisé en ce que l'agent de neutralisation précité est une amine tertiaire de formule IB dans laquelle :
- m est égal à 0 et
- l'un au moins de R1, R2, R5 et R6 représente un radical hydroxyalkyle ayant de 2 à 4 atomes de carbone.

7. Procédé selon la revendication 6, caractérisé en ce que l'agent de neutralisation précité est la N,N,N',N'-tétrakis(hydroxypropyl) éthylène diamine.

8. Procédé selon l'une quelconque des revendications 1 à 7, caractérisé en ce que la quantité d'agent de neutralisation utilisée à l'étape b précitée est suffisante pour permettre l'obtention d'un pH compris entre 5 et 9.

## Patentansprüche

1. Verfahren zur Herstellung eines Alkylpolysaccharids mit 6 bis 32 Kohlenstoffatomen in seiner Alkylkette der Art, welche einschließt:
a) Die Reaktion, in Gegenwart eines sauren Katalysators, zwischen einem Saccharid oder einer Saccharidquelle und
- entweder einem Fettalkohol mit einer Kohlenwasserstoffkette mit wenigstens 6 Kohlenstoffatomen,
- oder einem niederen Alkohol der Formel CₙH₂ₙ₊₁OH, worin n eine ganze Zahl zwischen 1 und 5 ist, und einem Fettalkohol mit einer Kohlenwasserstoffkette mit wenigstens 6 Kohlenstoffatomen;
b) Neutralisation des in Schritt a) verwandten Katalysators und
c) Entfernung überschüssigen Fettalkohols;
dadurch gekennzeichnet, daß das im vorgenannten Schritt b) verwandte Neutralisationsmittel ein tertiäres Amin der Formel 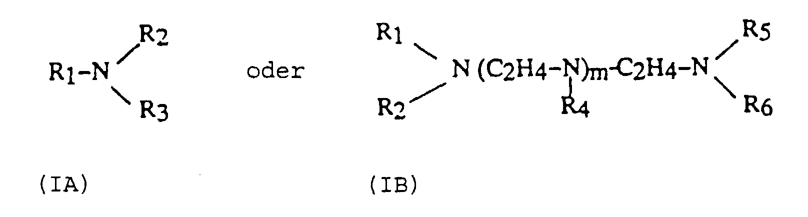 ist, worin
- m eine ganze Zahl zwischen 0 und 5 darstellt;
- R₁, R₂, R₃, R₄, R₅, R₆, gleich oder verschieden, unabhängig voneinander darstellen:
einen Alkylrest mit 1 bis 6 Kohlenstoffatomen;
einen Hydroxyalkylrest oder Dihydroxyalkylrest mit 2 bis 4 Kohlenstoffatomen;
einen Phenylrest;
einen Rest der Formel (CₙH₂ₙ-O)ₓH, worin n eine ganze Zahl zwischen 2 und 4 ist und x eine ganze Zahl zwischen 2 und 5;
- R₁R₂ zusammengenommen mit dem Stickstoffatom, an das sie gebunden sind, einen Heterocyklus darstellen, gesättigt oder nicht gesättigt, substituiert oder nicht substituiert, 5- bis 7-gliedrig und mit 1 bis 3 Heteroatomen, ausgewählt aus Sauerstoff, Stickstoff und Schwefel.

2. Verfahren nach Anspruch 1, dadurch gekennzeichnet, daß das vorgenannte Neutralisationsmittel ein tertiäres Amin der Formel IA ist, worin wenigstens ein Rest von R₁, R₂ und R₃ einen Hydroxyalkyl- oder Dihydroxyalkylrest mit 2 bis 4 Kohlenstoffatomen darstellt.

3. Verfahren nach Anspruch 2, dadurch gekennzeichnet, daß das Neutralisationsmittel Triethanolamin ist.

4. Verfahren nach Anspruch 1, dadurch gekennzeichnet, daß das vorgenannte Neutralisationsmittel ein heterozyklisches tertiäres Amin der Formel IA ist, worin R₁R₂ zusammengenommen mit dem Stickstoffatom, an das sie gebunden sind, einen aus Pyrrol, Pyrrolidon, Pyrrolidin, Piperidin und Morpholin ausgewählten Heterocyklus bilden, und R₃ einen Alkylrest mit 1 bis 6 Kohlenstoffatomen oder einen Hydroxyalkylrest mit 2 bis 4 Kohlenstoffatomen darstellt.

5. Verfahren nach Anspruch 4, dadurch gekennzeichnet, daß das vorgenannte Neutralisationsmittel aus N-Methylpyrrol, N-Methylpyrrolidon, N-Methylpyrrolidin, N-Methylpiperidin, N-Methylmorpholin und N-Hydroxyethylmorpholin ausgewählt wird.

6. Verfahren nach Anspruch 1, dadurch gekennzeichnet, daß das vorgenannte Neutralisationsmittel ein tertiäres Amin der Formel IB ist, worin
- m 0 ist und
- wenigstens ein Rest von R₁, R₂, R₅ und R₆ einen Hydroxyalkylrest mit 2 bis 4 Kohlenstoffatomen darstellt.

7. Verfahren nach Anspruch 6, dadurch gekennzeichnet, daß das vorgenannte Neutralisationsmittel N,N,N',N'-Tetrakis(hydroxypropyl)ethylendiamin ist.

8. Verfahren nach einem der Ansprüche 1 bis 7, dadurch gekennzeichnet, daß die Menge an in der vorgenannten Stufe b verwandtem Neutralisationsmittel ausreicht, einen pH-Wert zwischen 5 und 9 einzustellen.

## Claims

1. A process for the preparation of an alkylpolyoside containing from 6 to 32 carbon atoms in its alkyl chain, of the type comprising:
a) the reaction, in the presence of an acid catalyst, of a saccharide or a source of saccharide with
- either a fatty alcohol having a hydrocarbon chain containing at least 6 carbon atoms,
- or a lower alcohol of the formula CₙH₂ₙ₊₁OH, in which n is an integer between 1 and 5, and a fatty alcohol having a hydrocarbon chain containing at least 6 carbon atoms;
b) the neutralization of the catalyst used in step a); and
c) the removal of the excess fatty alcohol;
characterized in that the neutralizing agent used in step b) above is a tertiary amine of the formulae 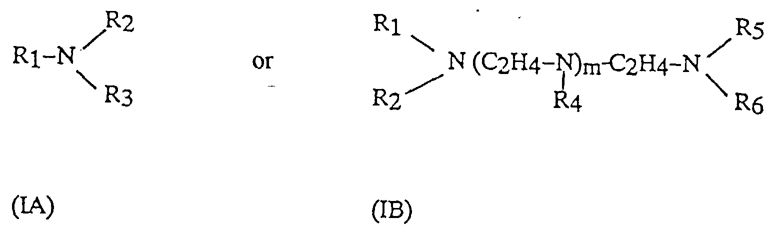 in which
- m is an integer between 0 and 5;
- R₁, R₂, R₃, R₄, R₅ and R₆, which are identical or different, are independently:
an alkyl radical having from 1 to 6 carbon atoms,
a hydroxyalkyl or dihydroxyalkyl radical having from 2 to 4 carbon atoms,
a phenyl radical,
a radical of the formula (CₙH₂ₙ-O)ₓH, in which n is an integer between 2 and 4 and x is an integer between 2 and 5,
- it is possible for R₁R₂, taken together with the nitrogen atom to which they are attached, to be a saturated or unsaturated, substituted or unsubstituted heterocycle having from 5 to 7 ring members and containing from 1 to 3 heteroatoms selected from oxygen, nitrogen and sulfur.

2. The process according to claim 1, characterized in that the above-mentioned neutralizing agent is a tertiary amine of formula IA in which at least one of R₁, R₂ and R₃ is a hydroxyalkyl or dihydroxyalkyl radical having from 2 to 4 carbon atoms.

3. The process according to claim 2, characterized in that the neutralizing agent is triethanolamine.

4. The process according to claim 1, characterized in that the above-mentioned neutralizing agent is a heterocyclic tertiary amine of formula IA in which R₁R₂, taken together with the nitrogen atom to which they are attached, form a heterocycle selected from pyrrole, pyrrolidone, pyrrolidine, piperidine and morpholine, and R₃ is an alkyl radical having from 1 to 6 carbon atoms or a hydroxyalkyl radical having from 2 to 4 carbon atoms.

5. The process according to claim 4, characterized in that the above-mentioned neutralizing agent is selected from N-methylpyrrole, N-methylpyrrolidone, N-methylpyrrolidine, N-methylpiperidine, N-methylmorpholine and N-hydroxyethylmorpholine.

6. The process according to claim 1, characterized in that the above-mentioned neutralizing agent is a tertiary amine of formula IB in which:
- m is equal to 0 and
- at least one of R₁, R₂, R₅ and R₆ is a hydroxyalkyl radical having from 2 to 4 carbon atoms.

7. The process according to claim 6, characterized in that the above-mentioned neutralizing agent is N,N,N',N'-tetrakis(hydroxypropyl)ethylenediamine.

8. The process according to any one of claims 1 to 7, characterized in that the amount of neutralizing agent used in step b above is sufficient to enable a pH of between 5 and 9 to be obtained.
